Europäisches Patentamt

European Patent Office

Office européen des brevets

(11) Veröffentlichungsnummer: **0 281 803**
**A1**

## (12) EUROPÄISCHE PATENTANMELDUNG

(21) Anmeldenummer: 88102268.5

(22) Anmeldetag: 17.02.88

(51) Int. Cl.⁴: **C12N 15/00** , A61K 39/245 ,
C07K 15/00 , G01N 33/569

(30) Priorität: 26.02.87 DE 3706233

(43) Veröffentlichungstag der Anmeldung:
14.09.88 Patentblatt 88/37

(84) Benannte Vertragsstaaten:
**AT BE CH DE FR GB IT LI LU NL SE**

(71) Anmelder: **BEHRINGWERKE**
**Aktiengesellschaft**
**Postfach 1140**
**D-3550 Marburg 1(DE)**

(72) Erfinder: **Müller-Lantzsch, Nikolaus, Prof.Dr.**
**Hausmattenweg 3**
**D-7801 Oberried(DE)**
Erfinder: **Nüblich, Micha**
**Rennweg 2**
**D-7800 Freiburg(DE)**
Erfinder: **Boos, Harald**
**Terlanerstrasse 2**
**D-7800 Freiburg(DE)**
Erfinder: **Schuy, Wilhelm, Dr.**
**Schliefelder Weg 6**
**D-5431 Obererbach(DE)**
Erfinder: **Bröker, Michael, Dr.**
**Lindenweg 16**
**D-3550 Marburg(DE)**

(74) Vertreter: **Meyer-Dulheuer, Karl-Hermann, Dr.**
**et al**
**HOECHST Aktiengesellschaft Zentrale**
**Patentabteilung Postfach 80 03 20**
**D-6230 Frankfurt/Main 80(DE)**

(54) **Immunogene Fusionsproteine, die Epstein-Barr-Virusproteine enthalten.**

(57) Gentechnisch erhaltene Fusionsproteine, die Anteile von Epstein-Barr-Virus(EBV)-Glykoprotein 350 oder 220 enthalten, eignen sich aufgrund ihrer Immunogenität zur Herstellung von Antikörpern oder Antiseren und zur Bestimmung von anti-EBV-Antikörper-Titern in humanen Seren. Die Antikörper und Fusionsproteine ermöglichen die Herstellung entsprechender Diagnostika.

## Immunogene Fusionsproteine, die Epstein-Barr-Virusproteine enthalten

Das Epstein-Barr-Virus (im folgenden EBV) ist ein lymphotropes, humanes Herpesvirus, das ein ätiologisches Agens der infektiösen Mononukleose ist und mit Nasopharyngealkarzinom, Burkitt-Lymphom und polyklonaler B-Zell-Proliferation bei Immundefekten assoziiert ist. Es hat außerdem die Eigenschaft, B-Lymphocyten in vitro immortalisieren zu können.

Ein Teil der gegen das EBV entwickelten Immunantwort ist gegen die beiden Glykoproteine gp 350 und gp 220 gerichtet (Müller-Lantzsch et al., Virology 102 (1980) 401-411). Es stellte sich heraus, daß diese beiden Hüllproteine im EBV-Genom überlappend codiert sind und auf dem Bam HI-L-Fragment lokalisiert sind (Beisel et al., J. Virology 54 (1985) 655-674).

Es wurde nun gefunden, daß gentechnisch hergestellte, unglykosylierte Fusionsproteine in der EBV-Serologie einsetzbar sind. Die Erfindung betrifft deshalb immunogene Fusionsproteine, die Anteile von EBV-gp 350 und gp 220 enthalten, ihr Einsatz als Antigen bei der Bestimmung von anti-EBV-Antikörper-Titern humaner Seren, ihre Verwendung zur Herstellung von Antiseren und Antikörpern sowie die so erhaltenen Antiseren und Antikörper. Die verschiedenen Aspekte der Erfindung sind in den Patentansprüchen definiert; bevorzugte Ausgestaltungen werden im folgenden näher erläutert.

Die DNA-Sequenz des EBV-Stammes B95-8 ist bekannt (Baer et al., Nature 310 (1984) 207-211). Das Bam HI-L-Fragment des EBV-Stammes M-ABA stimmt mit dieser veröffentlichten Sequenz überein (Polack et al., Gene 27 (1984) 279-288).

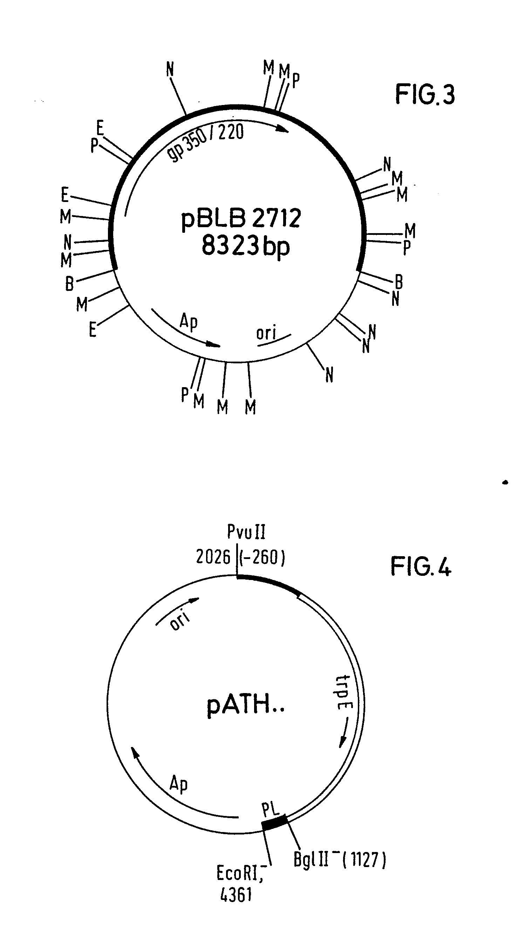

Der von Polack et al. beschriebene pHC79-Cosmid-Klon cM302-23 kann als Ausgangsmaterial für die gentechnische Herstellung der erfindungsgemäßen Fusionsproteine dienen, da dieser Klon das Bam HI-L-Fragment umfaßt (Fig. 3 von Polack et al.). Aus diesem Klon kann nach Schneiden mit Bam HI das L-Fragment in bekannter Weise isoliert werden. Selbstverständlich kann aus Ausgangsmaterial auch jeder beliebige andere Klon dienen, der dieses Fragment umfaßt. Auf Grund der Zuordnung in Tabelle 1 von Baer et al. ist die Gewinnung geeigneter Klone aus EBV-Genbanken ohne weiteres möglich.

Das Bam HI-L-Fragment kann dann in einen Vektor, beispielsweise pBR327, ligiert und in E. coli amplifiziert werden. Man gewinnt so die nötige DNA für Subklonierungen die zu den erfindungsgemäßen Fusionsproteinen führen.

Die erfindungsgemäßen Fusionsproteine können grundsätzlich mit beliebigen Expressionsvektoren erzeugt werden. Als vorteilhaft haben sich solche Vektoren herausgestellt, die von einem Protein des trp-Systems von E. coli Gebrauch machen, wie sie beispielsweise in der europäischen Patentanmeldung mit der Veröffentlichungsnummer 0 198 415 und der darin genannten Literatur beschrieben sind. Besonders vorteilhaft sind Vektoren, die für Fusionsproteine codieren, deren bakterieller Anteil sich vom E-Protein des trp-Operons von E. coli ableitet. Solche Vektoren sind schon zur Herstellung von Fusionsproteinen mit viralen Proteinanteilen benutzt worden (Spindler et al., J. Virology 49 (1984) 132-141). Als besonders zweckmäßig hat sich ein Vektorensystem herausgestellt, das sich aus dem großen PvuII-HindIII-Fragment bzw. PvuII-EcoRI-Fragment von pBR322, dem PvuII-BGIII-Fragment aus dem trp-Operon-Bereich von E. coli (Positionen -260 bis 1127) und einem der in der Tabelle 1 dargestellten Polylinker zusammensetzt. Diese Vektoren werden als pATHx bezeichnet, wobei x die in der Tabelle 1 genannte Kennziffer ist. In der Tabelle 1 wurde bei der Sequenz (angegeben ist jeweils

```
pATH
                SmaI        SacI        EcoRI         SacI        SmaI        BamHI       XbaI        SalI
  1  ATT GAG ATC CCC GGG CGA GCT CGA ATT CGA GCT CGC CCG GGG ATC CTC TAG AGT CGA CCT

                SmaI        BamHI       XbaI        SalI        PstI        HindIII     ClaI
  2  ATT GAG ATC CCC GGG GAT CCT CTA GAG TCG ACC TGC AGC CCA AGC TTA TCG ATG ATA AGC

                SmaI        EcoRI       SstI        SmaI        BamHI       XbaI        SalI        PstI
 10  ATT GAG ATC CCC CCG AAT TCT AGC TCG CCC GGG GAT CCT CTA GAG TCG ACC TGC AGC CCA

                SmaI        EcoRI       SstI        ClaI        SmaI        BamHI       XbaI        SalI        PstI    HindIII
 11  ATT GAG ATC CCC GAA TTC GAG CTC GCC CGG GGA TCC TCT AGA GTC GAC CTG CAG CCC AAG

                PstI        HindIII     ClaI                    EcoRI
  1  GCA GCC CAA GCT TAT CGA TGA TAA GCT GTC AAA CAT GAG AAT TAA TTC TTG AAG ACG AAA

                                        ClaI
                HindIII
  2  AGT TGT CAA ACA TGA GAA TTA ATT CTT GAA GAC GAA

                ClaI
 10  AGC TTA TCG ATG ATA

                HindIII
 11  CCT ATC GAT GAT
```

nur der codierende Strang) CCC GGG nur das Enzym "SmaI" angegeben; bekanntlich wird diese Sequenz auch von XmaI erkannt (wenn auch in anderer Weise geschnitten). Diese "SmaI" bezeichneten Stellen in den Polylinkersequenzen sollen somit auch die Schnittstelle für XmaI bezeichnen. Überraschenderweise zeigt es sich, daß humane Seren gegen den bakteriellen Anteil dieser Fusionsproteine keine Antikörper enthalten.

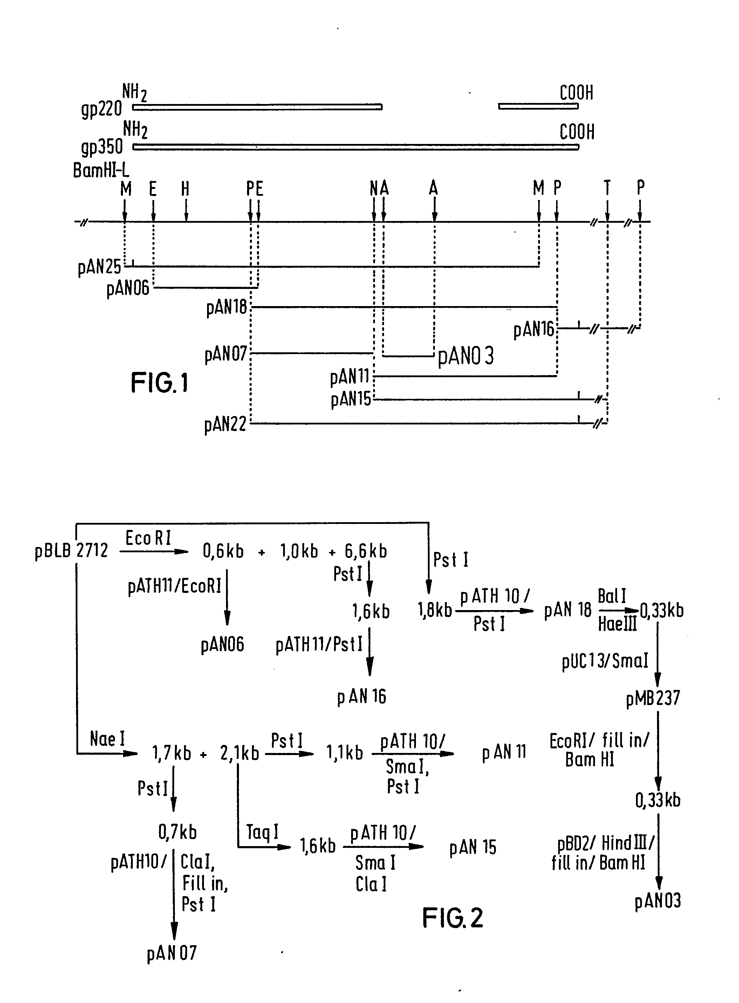

Die für trpE-Fusionsproteine codierenden Konstrukte wurden als pAN .. bezeichnet, wobei die Kennziffer annähernd die Größe des Inserts in 0,1 kb angibt. Die Figur 1 zeigt eine Übersicht über die ausgewählten Fragmente und deren Lage im gp 350/gp 220-Leseraster. Für die Restriktionsschnittstellen wurden die folgenden Abkürzungen verwendet:

A = HaeIII

E = EcoRI

H = HindIII

M = MaeI

N = NaeI

P = PstI

T = TaqI

Figur 2 zeigt die Klonierungsstrategie zur Herstellung der Konstrukte pAN .., wobei pBLB 2712 das Ausgangsplasmid bezeichnet, das durch Insertion des BamHI-L-Fragments in die BamHI-Schnittstelle von pBR 327 erhalten wurde. Auf die Wiedergabe der Konstruktion der Plasmide pAN 22 und pAN 25 wurde verzichtet.

Werden die Plasmide pAN .. in E. coli transformiert und mit Indolacrylsäure (IAA) induziert, so werden die codierten Fusionsproteine exprimiert. Nach Isolierung können die Fusionsproteine mit Hilfe von humanen Standardseren, deren Antikörpertiter gegen virale Proteine bekannt ist, auf Immunoreaktion geprüft werden.

Die immunogegen Fusionsproteine können dann erfindungsgemäß in diagnostischen Verfahren als Antigene zur Bestimmung von anti-EBV-Titern in Seren oder zur Herstellung hochspezifischer Antiseren bzw. Antikörper eingesetzt werden, die dann in üblichen diagnostischen Agenzien Verwendung finden. Die Erfindung betrifft deshalb auch diagnostische Mittel, die solche Antikörper oder Fusionsproteine enthalten.

In den folgenden Beispielen wird die Erfindung näher erläutert.


Beispiel 1: Herstellung des Plasmids pBLB2712

Als Ausgangsmaterial für die Klonierung des BamHI-L-Fragments diente der Cosmid-Klon cM 302 (Polack et al., a.a.O.), der als Insert 29 kb aus der mittleren DNA-Region des EBV-Stammes M-ABA enthält, die die BamHI-Fragmente S, L, E, f, e, Z, R und Teile von M und K umfaßt. Dieser Cosmid-Klon wurde mit BamHI gespalten, das BamHI-L-Fragment isoliert und in die BamHI-Schnittstelle des Vektors pBR327 ligiert. Nach Transformation des E. coli-Stammes HB 101 mit dem Ligierungsansatz wurden aus 12 Kolonien die Plasmide isoliert. Durch Restriktionsanalyse wurden diejenigen Klone identifiziert, die das L-Fragment in der gewünschten Orientierung enthielten (Figur 3, Abkürzungen wie in Figur 1). Das Plasmid pBLB2712 wurde präparativ angereichert und diente dann als Ausgangs-DNA für alle weiteren Subklonierungen.


Beispiel 2: Expressionsvektoren pATH ..

Das große PvuII-EcoRI-pBR322-Fragment mit dem pBR322-Replikationsursprung wurde mit dem 1,4 kb PvuII-BglII-Fragment aus dem E. coli-trp-Operon mit dem E-Fragment ligiert und die überstehenden Enden durch Auffüllen stumpfendig gemacht. Anschließend wurde unter "blunt end"-Bedingungen einer der in Tabelle 1 genannten Polylinker einligiert.

Die Figur 4 zeigt die Konstruktion, wobei die dünne Linie den pBR322-Anteil darstellt unf "PL" für Polylinker steht. Die Positionsziffern in Klammern beziehen sich auf das E. coli-trp-Operon, die anderen auf die pBR322-Positionen.


Beispiel 3: Subklonierungen

a) pAN 06

Das Plasmid pBLB2712 wird mit EcoRI geschnitten, wobei drei Fragmente der Größe 0,6, 1,0 und 6,6 kb erhalten werden. Das 0,6 kb-Fragment wird direkt in die EcoRI-Schnittstelle des Vektors pATH 11 ligiert, wobei das Plasmid pAN 06 erhalten wird. Nach Transformation von E. coli HB 101 wurden die Konstrukte mit der richtigen Orientierung des Inserts durch Spaltung mit PstI erkannt.

b) pAN 16

Das bei der vorstehen beschriebenen Spaltung erhaltene Fragment der Größe 6,6 kb wird mit PstI gespalten, wobei u.a. zwei Fragmente der Größe 1,6 und 1,8 kb erhalten werden. Das 1,6 kb große Fragment wird direkt in den mit PstI gespaltenen Vektor pATH 11 ligiert. Nach Transformation von E. coli HB 101 werden durch Restriktionsanalyse die Klone mit der gewünschten Orientierung identifiziert, die als pAN 16 bezeichnet werden.

c) pAN 18

Das bei der Spaltung von pBLB2712 mit PstI erhaltene Fragment von 1,8 kb Größe wird in den mit PstI geschnittenen Vektor pATH 10 ligiert. Transformation in E. coli HB 101 und Restriktionsanalyse ergibt dit gewünschten Klone, die als pAN 18 bezeichnet werden.

d) pAN 07

Das Plasmid pBLB 2712 wird mit NaeI geschnitten, wobei zwei Fragmente der Größe 1,7 und 2,1 kb entstehen. Das kleinere Fragment wird isoliert und mit PstI nachgeschnitten, wobei ein 0,7 kb großes Fragment erhalten wird. Der Vektor pATH 10 wird zunächst mit ClaI geöffnet, die überstehenden Enden aufgefüllt und dann mit PstI nachgeschnitten, worauf das 0,7 kb große Fragment hineinligiert wird. Nach Transformation in E. coli HB 101 werden die gewünschten Klone, die als pAN 07 bezeichnet werden, identifiziert.

e) pAN 11

Das bei der Spaltung von pBLB2712 mit NaeI erhaltene 2,1 kb große Fragment wird mit PstI umgesetzt und das 1,1 kb große Fragment isoliert. Der Vektor pATH 10 wird mit SmaI und PstI geöffnet und das linearisierte Plasmid mit dem 1,1 kb großen Fragment ligiert. Nach Transformation von E. coli HB 101 identifiziert man durch Restriktionsanalyse die gewünschten Konstrukte, die als pAN 11 bezeichnet werden.

f) pAN 15

Das bei der Spaltung von pBLB2712 mit NaeI erhaltene 2,1 kb große Fragment wird mit TaqI umgesetzt und das 1,6 kb große Fragment isoliert. Der Vektor pATH10 wird mit SmaI und ClaI umgesetzt und das linearisierte Plasmid mit dem 1,6 kb großen Fragment ligiert. Nach Transformation in E. coli HB 101 werden durch Restriktionsanalyse die gewünschten Klone identifiziert, die als pAN 15 bezeichnet werden.

g) pAN 03

Das bei der Spaltung von pAN 18 mit BalI erhaltene 1230 bp große Fragment wurde mit HaeIII umgesetzt und das 330 bp große Fragment isoliert. Um dieses DNA-Fragment in Form eines Fusionsproteins exprimieren zu können, wurde die DNA zunächst in pUC13, das mit SmaI verdaut worden war, subkloniert. Das erhaltene Plasmid pMB237, in dem der EBV-kodierende DNA-Abschnitt in der gleichen Orientierung wie die durch pUC13 kodierte Sequenz des $\beta$-Galactosidase-$\alpha$-Peptids vorliegt, wurde mit EcoRI verdaut und die überstehenden Enden durch Polymerase (Klenow-Fragment) aufgefüllt. Anschließend wurde das Plasmid mit BamHI verdaut, das 330 bpgroße BamHI/EcoRI-Fragment isoliert und in pBD2 (Bröker, Gene Anal.Techn.3 (1986), 53-57) ligiert. Der Vektor pBD2 war vorher mit HindIII geöffnet, mit Polymerase I (Klenow-Fragment) behandelt und anschließend mit BamHI verdaut worden. In dem neu entstandenen Vektor pAN 03 ist der gp350-Anteil von Aminosäure 503-613 an eine verkürzte Form der $\beta$-Galactosidase von 375 Aminosäuren fusioniert. In E. coli BMH71-18 und anderen lacI$^q$-tragenden Stämmen ist das durch pAN 03 kodierte Fusionsprotein nach Zugabe von IPTG stabil zu exprimieren.

Beispiel 4: Identifizierung der Fusionsproteine im "Immunoblot"

Zur Identifizierung der Fusionsproteine wurde ein EBV-spezifisches humanes Serum (Serum BG) eingesetzt, das die folgenden Titer (EBV-Immunfluoreszenz) aufwies:

αVCA     4000

αEA      512

αEBNA    320

αtrpE    -

α = anti

VCA = Virus-Capsid-Antigen

EA = Frühes Antigen ("early antigen")

EBNA = EBV-spezifisches nukleäres Antigen

trpE = bakterielle Anthranilatsynthase

Mit Hilfe des Serums BG kann die Expressionskinetik verfolgt werden: Vor der Induktion ist weder trpE noch ein Fusionsprotein wahrnembar. Nach einer Fermentation bis zu 20 Stunden ab Induktion steigt die Menge an trpE und an Fusionsproteinen mit der Dauer der Induktion an. Bei den Fusionsproteinen überlagert sich - steigend mit der Größe des EBV-Anteils - ein Abbau des Proteins.

Der Abbau der Fusionsproteine verläuft im allgemeinen nur bis zu einer bestimmten Größe, beispielsweise das von pAN 25 codierte Protein von 150 kD wird bis zu 85 kD abgebaut, das von pAN 18 codierte Protein von 135 kD bis zu 65 kD, das von pAN 06 codierte Protein von 60 kD bis zu 50 kD, während das von pAN 16 codierte Protein von 40 kD stabil ist. Die angegebenen Molgewichte wurden im "blot" durch Coelektrophorese von Markerproteinen ermittelt. Sie sind jeweils um etwa 30% höher als die auf Grund der Aminosäurezusammensetzung berechneten Molgewichte. Diese höheren Molgewichte lassen sich auf ein verändertes Laufverhalten, bedingt durch den hohen Prolinanteil, zurückführen.

Beispiel 5: Erkennung der Fusionsproteine durch spezielle Humanseren

Die Auswahl an Seren (Tab. 2) umfaßte solche ohne αVCA-Titer (Seren 1 und 2) und andere mit αVCA-IgG-Titer, aber ohne αVCA-IgA-Titer (Seren 3 und 4). Seren 5-12 hatten sowohl αVCA-IgG-als auch αVCA-IgA-Titer; hiervon stammten die Seren 5-9 von Nasopharyngealkarzinom-(NPC-) Patienten, die Seren 10-12 von anderen Patienten. Die Titer der einzelnen Seren waren in der Immunfluoreszenz bestimmt worden. Die Seren 10-12 konnten auf Grund zu geringer vorhandener Mengen nicht mit allen Fusionsproteinen getestet werden.

Tabelle 2

| Serum Nr. | Diagnose | αVCA-IgG | αVCA-IgA |
|---|---|---|---|
| 1 | | - | - |
| 2 | | - | - |
| 3 | | 64 | - |
| 4 | | 256 | - |
| 5 | NPC | 256 | 32 |
| 6 | NPC | 2048 | 16 |
| 7 | NPC | 4000 | 256 |
| 8 | NPC | 1024 | 1024 |
| 9 | NPC | 2048 | 2048 |
| 10 | CLL | 1024 | 256 |
| 11 | Leukämie | 8000 | 64 |
| 12 | IM | 1024 | 64 |

CLL = Chronische lymphatische Leukämie;
IM = Infektiöse Mononukleose

Beim Test auf Gesamtzellextrakte aus dem E. ccli-Stamm C 600, der mit dem Vektor pATH 1 transformiert und zwei Stunden unter Induktionsbedingungen kultiviert worden war, wurde festgestellt, daß die Seren zwar einen IgA-Titer gegen verschiedene bakterielle Proteine besitzen, jedoch nicht gegen den im Molekulargewichtsbereich von 37 kD laufenden trpE-Anteil (Anthranilatsynthase). Eine Erkennung von Fusionsproteinen auf Grund einer αtrpE-IgA-Reaktivität kann damit praktisch ausgeschlossen werden. Bei den Seren 1 bis 3 und 5 bis 9 existieren keine IgA-Titer gegen bakterielle Proteine, Serum 4 zeigt einen IgA-Titer gegen ein Protein mit dem Molgewicht 60 kD und auch die Seren 10 bis 12 weisen IgA-Antikörper gegen bakterielle Proteine auf.

a) pAN 06

Dieses 60 kD große Fusionsprotein enthält die Aminosäuren 32 bis 245 von gp 350. Das Fusionsprotein wird im IgG-Test von den Seren 6 bis 9 und im IgA-Test von den Seren 5, 6 und 8 erkannt.

b) pAN 16

Das 40 kD große Fusionsprotein umfaßt die Aminosäuren 865 bis 907 von gp 350, also die C-terminalen 43 Aminosäuren. Es handelt sich hierbei um den α-helikalen, hydrophoben "Membrananker" und einen kurzen Bereich innerhalb der Lipidhülle. Trotzdem enthalten die Seren Antikörper-Titer gegen dieses Fusionsprotein - möglicherweise hervorgegangen nach Lyse infizierter Zellen oder viraler Hüllen durch Entstehen von "inside-out"-Vesikeln. So wird dieses Fusionsprotein, das sich durch seine große Stabilität auszeichnet, gut durch das Serum Nr. 9 erkannt.

c) pAN 18

Das Fusionsprotein von 135 kD umfaßt mit den Aminosäuren 232 bis 866 den größten Teil von gp 350. Es ist das im Immuntest am besten erkannte Protein, sowohl im IgG-als auch im IgA-Test. Wegen der hohen Empfindlichkeit ist ein Nachweis auch noch in einer Verdünnung von 1 : 100 möglich.

Wegen der unbefriedigenden Stabilität erfolgte eine Subklonierung zu pAN 07 und pAN 11.

d) pAN 07

Das 78 kD große Fusionsprotein umfaßt die Aminosäuren 232 bis 479. Es zeichnet sich durch Stabilität aus, wohl weil es keine "repeats" umfaßt. Im IgA-Test wird das Fusionsprotein von den Seren 7, 9, 11 und 12 gut erkannt.

e) pAN 11

Das 79 kD große Fusionsprotein umfaßt die Aminosäuren 480 bis 866 von gp 350 und damit die "repeats", was wohl für seine relativ geringe Stabilität, verantwortlich ist.

f) pAN 15

Das 84 kD große Fusionsprotein umfaßt die Aminosäuren 480 bis 907 von gp 350. Es zeichnet sich durch hohe Stabilität aus, obwohl es den Aminosäurebereich des von pAN 11 codierten Fusionsproteins einschließt. Offenbar wird das Protein durch den hydrophoben Membrananker (21 Aminosäuren) und den kurzen Proteinbereich innerhalb der Lipidhülle (20 Aminosäuren) stabilisiert.

Das Fusionsprotein wird von allen Seren im IgA-und IgG-Test erkannt und reagiert auch mit allen Seren im IgG-Test.

Beispiel 6: Erkennung der Fusionsproteine im "Enzyme-Linked Immunosorbent-Assay" (ELISA)

Zur Identifizierung der Fusionsproteine wurde ein EBV-spezifisches humanes Serum (Serum S262) eingesetzt, das einen Titer von 1:5000 gegen Gesamtvirus aus Zellkultur aufwies.

Die Tabelle 3 gibt die Ergebnisse wieder. Das Kontrollantigen pATH besaß einen Absolutwert von 0,49 -1,0.

Für den ELISA wurden ungereinigte Proteinpräparationen (Bakterienlysate) der genannten Klone (siehe Tab. 3) auf eine Mikrotitrationsplatte beschichtet.

Nachgewiesen wurde spezifische IgG. Die durch pAN 07, pAN 15 und pAN 18 exprimierten Protein werden spezifisch erkannt.

Die hohen Hintergrundreaktionen, wie sie durch das Kontrollantigen angezeigt werden, beruhen auf der Art der Präparation. Nicht so stark schwankende Extinktionsdifferenzen sind durch gezielte Reinigungsverfahren zu erhalten.

Beispiel 7: Einengung der immunologisch relevanten Aminosäuresequenzen von gp 350 (Fig. 1)

Das Fusionsprotein pAN 03 zeigte im ELISA, durchgeführt wie im Beispiel 6, keine spezifische Reaktion.

Getestet wurde einerseits das Serum S262 auf spezifische IgG-Antikörper und andererseits ein Mononukleose-Serum auf IgM-Antikörper. Da der untersuchte Bereich ein Subfragment von pAN 18 darstellt, ist zu vermuten, daß außerhalb des durch pAN 03 codierten Bereichs anderen Sequenzen, wie sie z. B. in pAN 11 und pAN 15 vorhanden sind, diagnostische Relevanz besitzen.

Dennoch besteht die Möglichkeit, daß der durch pAN 03 codierte Bereich des gp350, z. B. auf Grund sterischer Erfordernisse, ein immunologisch relevantes Epitop enthält, zumal es im "Western blot" mit Patientenseren spezifisch reagiert.

Tabelle 3

| Antigen<br>(Fusionsprotein) | pAN 07 | pAN 15 | pAN 18 |
|---|---|---|---|
| **Extinktionsdifferenz**<br>Antigen-<br>Kontrollantigen<br>(gemessen bei 450 nm) | 0,12 bis<br>0,52 | 0,17 bis<br>0,24 | 0,11 bis<br>0,38 |

Beispiel 8: Verwendung von gp 350 für Immunisierungszwecke

Stellvertretend für gp 350 wurde ein Fragment dieses Proteins, das Fusionsprotein pAN 03, zu Immunisierungen eingesetzt (4 x etwa 100 µg subkutan).

Zwei Kaninchen zeigten nach Abschluß der Immunisierung einen Antikörpertiter von 1:80 gegen eine Virusantigenpräparation aus der Zellkultur. Der Titer wurde in einem "Enzyme-Linked Immunosorbent Assay" (ELISA) bestimmt. Das Virus aus der Zellkultur war dazu auf die Mikrotitrationsplatte beschichtet worden. Die spezifischen Kaninchenantikörper wurden mit einen Anti-Kaninchen IgG/POD-Konjugat detektiert.

Hiermit ist gezeigt, daß in E. coli hergestellte Antigene (Fusionsproteine) in der Lage sind, die Produktion von Antikörpern zu induzieren. Dies bedeutet eine Verwendbarkeit für die Herstellung von Impfstoffen, die authentisches Virusprotein erkennen.

Weiterhin ist gezeigt, daß mit Seren, die durch Immunisierung mit rekombinanten EBV-Antigenen erhalten wurden, auch ein Virusnachweis durchgeführt werden kann.

## Ansprüche

1. Immunogene Fusionsproteine, die Anteile von Epstein-Barr-Virus-Glykoprotein (EBV-gp) 350 oder 220 enthalten.

2. Fusionsproteine nach Anspruch 1, gekennzeichnet durch die Aminosäuresequenzen 32-245, 232-479, 232-866, 480-866, 480-907, 503-613 oder 865-907 von EBV-gp 350.

3. Fusionsproteine nach Anspruch 1 oder 2, gekennzeichnet durch einen bakteriellen Anteil aus dem E-Protein des trp-Operons von E. coli.

4. Fusionsproteine nach Anspruch 1 oder 2, gekennzeichnet durch einen bakteriellen Anteil aus der β-Galaktosidase von E. coli.

5. Verwendung der Fusionsproteine nach Anspruch 1, 2, 3 oder 4 zur Herstellung von Antikörpern bzw. Antiseren.

6. Verwendung der Fusionsproteine nach Anspruch 1, 2, 3 oder 4 zur Herstellung von Impfstoffen für die passive und aktive Immunisierung.

7. Verwendung der Fusionsproteine nach Anspruch 1, 2, 3 oder 4 zur Bestimmung von anti-EBV-Antikörper-Titern in humanen Seren.

8. Antiseren und Antikörper, erhalten durch Immunisieren mit einem Fusionsprotein nach Anspruch 1, 2, 3 oder 4.

9. Diagnostikum, enthaltend einen Antikörper nach Anspruch 8.

10. Diagnostikum, enthaltend ein Fusionsprotein nach Anspruch 1, 2, 3 oder 4.

gp220 NH₂ ⟶ ... COOH

gp350 NH₂ ⟶ ... COOH

BamHI-L

M  E  H  PE  NA  A  M P  T  P

pAN25

pAN06

pAN18

pAN16

pAN07

pAN03

pAN11

pAN15

pAN22

## FIG.1

pBLB 2712 —EcoRI→ 0,6kb + 1,0kb + 6,6kb | PstI

pATH11/EcoRI ↓          PstI ↓

pAN06                    1,6kb      1,8kb —pATH 10/PstI→ pAN 18 —BalI/HaeIII→ 0,33kb

pATH11/PstI ↓                                           pUC13/SmaI ↓

pAN 16                                                  pMB237

                                                        EcoRI/ fill in/ BamHI ↓

pBLB 2712 —NaeI→ 1,7kb + 2,1kb —PstI→ 1,1kb —pATH 10/SmaI, PstI→ pAN 11     0,33kb

PstI ↓                                                                     pBD2/ HindIII/ fill in/ BamHI ↓

0,7kb          TaqI → 1,6kb —pATH 10/SmaI ClaI→ pAN 15                      pAN03

pATH10/ ClaI, Fill in, PstI ↓

pAN 07

## FIG.2

FIG.3

FIG.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 151 079 (THE UNIVERSITY OF CHICAGO)<br>* Beispiel 4 *<br>--- | 1,2,4-10 | C 12 N 15/00<br>A 61 K 39/245<br>C 07 K 15/00<br>G 01 N 33/569 |
| X | EP-A-0 163 573 (MERCK & CO. INC.)<br>* Beispiel 2 *<br>--- | 1,2,4-10 | |
| X | EP-A-0 173 254 (H.J. WOLF)<br>* Beispiele 14-19 *<br>--- | 1,2,4-10 | |
| A | WO-A-8 402 847 (AMGEN)<br>* Seite 53, Zeilen 33-37 *<br>----- | 3 | |

RECHERCHIERTE
SACHGEBIETE (Int. Cl.4)

C 12 N
C 12 P
A 61 K

Der vorliegende Recherchenbericht wurde für alle Patentansprüche erstellt

| Recherchenort | Abschlußdatum der Recherche | Prüfer |
|---|---|---|
| DEN HAAG | 16-06-1988 | CUPIDO M. |

EPO FORM 1503 03.82 (P0403)

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes
    Dokument